# EUROPEAN PATENT SPECIFICATION

(11) **EP 2 934 456 B1**
(45) Date of publication and mention of the grant of the patent: **02.03.2016**
(21) Application number: 13795756.9
(22) Date of filing: 26.11.2013
(51) Int. Cl.: A61Q 1/02, A61Q 1/06, A61Q 5/02, A61Q 5/12, A61Q 9/02, A61Q 15/00, A61Q 19/10, A61K 8/34, A61K 8/365, A61K 8/37, A61Q 17/04, A61K 8/67, A61Q 19/00

(54) **EUTECTIC MIXTURES IN PERSONAL CARE COMPOSITIONS**
EUTEKTISCH MISCHUNGEN IN KÖRPERPFLEGEZUSAMMENSETZUNGEN
MÉLANGES EUTECTIQUES DANS DES COMPOSITIONS DE SOINS PERSONNELS

(30) Priority: 20.12.2012 WO PCT/CN2012/087071; 25.01.2013 EP 13152644
(43) Date of publication of application: 28.10.2015
(73) Proprietor: Unilever N.V., 3013 AL Rotterdam (NL); Unilever PLC, London, Greater London EC4Y 0DY (GB)
(72) Inventor: KHOSHDEL, Ezat, Bebington Wirral Merseyside CH63 3JW (GB); SCHUMM, Stephan, Georg, NL-3133 AT Vlaardingen (NL); YAO, Yin-fang, Shanghai 200335 (CN); ZHANG, Qiqing, Shanghai 200335 (CN)
(74) Representative: Tansley, Sally Elizabeth
(86) International application number: PCT/EP2013/074687
(87) International publication number: WO 2014/095255

(56) References cited:
- EP-A1- 2 174 642
- WO-A1-2012/061991
- US-A1- 2004 018 954
- US-A1- 2010 093 682
- MATUO H ET AL: "Eutectic transformation in mixed monolayers of long normal chain fatty acids with 12-hydroxyoctadecanoic acid", CHEMISTRY AND PHYSICS OF LIPIDS, LIMERICK, IR, vol. 29, no. 1, 1 August 1981 (1981-08-01) , pages 55-67, XP025197949, ISSN: 0009-3084, DOI: 10.1016/0009-3084(81)90048-7 [retrieved on 1981-08-01]
- FIALA S ET AL: "A fundamental investigation into the effects of eutectic formation on transmembrane transport", INTERNATIONAL JOURNAL OF PHARMACEUTICS, ELSEVIER BV, NL, vol. 393, no. 1-2, 30 June 2010 (2010-06-30), pages 68-73, XP027065372, ISSN: 0378-5173, DOI: 10.1016/J.IJPHARM.2010.04.001 [retrieved on 2010-04-09]
- BENSON H A E: "TRANSDERMAL DRUG DELIVERY: PENETRATION ENHANCEMENT TECHNIQUES", CURRENT DRUG DELIVERY, BENTHAM SCIENCE PUBLISHERS, HILVERSUM, NL, vol. 2, no. 1, 1 January 2005 (2005-01-01) , pages 23-33, XP008049099, ISSN: 1567-2018, DOI: 10.2174/1567201052772915

## Description

### Field of the invention

The present invention relates to eutectic mixtures of L-menthol, L-menthyl lactate and 12-hydroxystearic acid; and personal care compositions comprising such eutectic mixtures.

### Background of the invention

12-hydroxystearic acid (hereinafter "12-HSA") is reported to have a wide variety of beneficial cosmetic effects on skin. It is a known PPAR-alpha (peroxisome proliferator activated receptors sub-type alpha) activator, skin lightening agent, and a sebum secretion inhibitor. See e.g. Alaluf et al. US 6, 423, 325, Mayes et al. US 6, 713, 051, WO2006/056283 (Hindustan Lever), Madison US 2009/0317341, Minami et al. US 6, 197, 343, Granger et al. US2004/0043044.

As such, cosmetic products containing 12-HSA are highly desirable.

JP 09-048962 describes the use of 12-HSA or its salt as an effective constituent of a solidification inhibitor, to inhibit solidification of a liquid detergent or a liquid cosmetic; all the examples containing a fully neutralized salt of 12-HSA. 12-HSA has traditionally been used as gelling agent e.g. in lipsticks and antiperspirant compositions. See also EP 0129528, US 6680285, Abbas et al. US 6, 680, 285, Tanner et al. US 5, 759, 524, WO95/31961 (Procter &. Gamble), Kawa et al., US 2004/0044078 (describing the use of 12-HSA to increase viscosity of cosmetic compositions), and JP 2010/138,110.

Unfortunately, 12-HSA is a solid having a melting point of about 72 to 77 degrees Celsius, has no water solubility and limited oil solubility. Salts of 12-HSA are only marginally more water-soluble. Because of the high melting temperature it is difficult to formulate in a personal care composition, often requiring elevated temperatures or solvents. Lowering the 12-HSA melting point or at least have it (partially) available as a non-solid may be desirable as this may e.g. influence the bioavailability or ease of use of 12-HSA or personal care compositions comprising 12-HSA. Another drawback associated with the high melting point of 12-HSA is that it may re-crystallize upon storage after being incorporated in a personal care product in non-solid form like e.g. in a solvent. Such re-crystallization may cause the undesired gellation of such personal care products thereby destroying the texture thereof.

The present inventors have identified a need for alternative methods to incorporate 12-HSA in personal care products that at least in part do not have one or more of the aforementioned drawbacks.

### Summary of the invention

The present inventors have found that certain mixtures comprising 12-HSA have a lower melting point than 12-HSA itself.

Accordingly, the invention relates to an active eutectic mixture of L-menthol, L-menthyl lactate and 12-hydroxystearic acid.

The invention further relates to a personal care composition comprising
i) the active eutectic mixture according the invention, and
ii) a cosmetically acceptable carrier.

In another aspect the invention relates to a method for preparing the personal care composition according to the invention comprising the step of mixing the preformed active eutectic mixture according to the invention of L-menthol, L-menthyl lactate and 12-hydroxystearic acid with part or all of the remaining ingredients of the personal care composition.

The invention also relates to a personal care composition obtainable by the method of the invention.

The invention further relates to a method of applying to human skin the personal care composition according to the present invention.

### Brief description of the figures

Certain embodiments of the invention will be described with reference to the figures in which:
Figure 1 shows DSC curves demonstrating the thermal stability behavior of a composition containing a preformed eutectic mixture of menthol-menthyl lactate-12 HSA (PCC1 - top curve) and a composition without menthol or menthyl lactate (PCC2 - bottom curve); and
Figure 2 shows DSC curves demonstrating the thermal stability behavior of a composition containing a preformed eutectic mixture of menthol-menthyl lactate-12 HSA (PCC1 - top curve) and a composition where menthol, menthyl lactate and 12 HSA were separately added (PCC3 - bottom curve).

### Detailed description of the invention

All amounts are by weight of the total composition, unless otherwise specified. Room temperature is defined as a temperature of about 20 degrees Celsius.

### Eutectic mixtures

A eutectic system is a mixture of chemical compounds or elements that has the lowest melting point of any other mixture made up of the same ingredients and wherein the composition of the liquid and the solid that are in equilibrium at this lowest melting temperature are the same. This composition is known as the "eutectic composition" and the corresponding melting temperature as the "eutectic temperature".

The present invention relates to the mixture of three components that gives rise to a ternary eutectic composition, i.e. a ternary composition that has a lower melting point than any other composition made of the three components, including the eutectic composition of any binary mixture of any two of the components.

For the purpose of the present invention an "active eutectic mixture" is defined as any ternary mixture of the three components of the present invention that is in the liquid state above a given melting temperature.

The active eutectic mixtures of the invention are mixtures of L-menthol, L-menthyl lactate and 12-hydroxystearic acid, wherein the weight ratio of L-menthol to L-menthyl lactate is from 2:8 to 9:1; the concentration of 12-hydroxystearic acid calculated on total weight of the eutectic mixture is from 0.1 to 17 wt%; and wherein the eutectic mixture has a melting point below 40 degrees Celsius.

The L-menthol and L-menthyl lactate are thus present in a weight ratio from 2:8 to 9:1. Preferably the weight ratio of L-menthol to L-menthyl lactate is from 2:8 to 8:2, more preferably from 3:7 to 7:3, even more preferably from 3:7 to 6:4, still more preferably from 3:7 to 5:5 and even still more preferably from 4:6 to 5:5.

The concentration of 12-hydroxystearic acid calculated on total weight of the eutectic mixture is from 0.1 to 17 wt%. Preferably the concentration of 12-hydroxystearic acid calculated on total weight of the eutectic mixture is from 0.1 to 15 wt%, more preferably from 0.1 to 10 wt%, even more preferably from 0.2 to 5 wt% and still more preferably from 0.5 to 3 wt%.

For example a preferred active eutectic mixture of 39.6 wt% L-menthol, 59.4 wt% L-menthyl lactate and 1.0 wt% 12-HSA corresponds to 99 wt% (L-menthol + L-menthyl lactate) in a weight ratio of 4:6 and a concentration of 12-hydroxystearic acid of 1 wt% with respect to the total weight of the three components together.

Preferred eutectic mixtures have a L-menthol and L-menthyl lactate weight ratio of 3:7 to 6:4 and a concentration of 12-hydroxystearic acid of 1 wt%, like for example a L-menthol and L-menthyl lactate weight ratio of 3:7 and a concentration of 12-hydroxystearic acid of 1 wt%; and a L-menthol and L-menthyl lactate weight ratio of 4:6 and a concentration of 12-hydroxystearic acid of 1 wt%.

Also preferred are eutectic mixtures that have a L-menthol and L-menthyl lactate weight ratio of 3:7 to 6:4 and a concentration of 12-hydroxystearic acid of 3 wt%, like for example a L-menthol and L-menthyl lactate weight ratio of 5:5 and a concentration of 12-hydroxystearic acid of 3 wt%; and a L-menthol and L-menthyl lactate weight ratio of 6:4 and a concentration of 12-hydroxystearic acid of 3 wt%.

The melting point of the eutectic mixture according to the invention is below 40 degrees Celsius. Preferably the mixture has a melting point below 37 degrees Celsius, more preferably below 30 degrees Celsius, even more preferably below 25 degrees Celsius, still more preferably below 20 degrees Celsius, even still more preferably below 15 degrees Celsius and more so preferably below 10 degrees Celsius. For example the eutectic mixture may have a melting point below 5 or 0 (zero) degrees Celsius. Preferably the eutectic mixture has a melting point above minus seventy (-70) degrees Celsius.

The amounts of the three ingredients in the ternary eutectic mixture of the invention will determine the melting of said mixture and can be suitably determined using well known methods as described in the example section of this specification.

Preferred eutectic mixtures have a L-menthol and L-menthyl lactate weight ratio of 3:7 to 7:3; a concentration of 12-hydroxystearic acid from 0.1 to 10 wt%; and a melting point below 35 degrees Celsius. An example of such a preferred eutectic mixture is a eutectic mixture that has a L-menthol and L-menthyl lactate weight ratio of 3:7 to 7:3; a concentration of 12-hydroxystearic acid from 0.1 to 10 wt%; and a melting point below 30 degrees Celsius. Another example of such a preferred eutectic mixture is a eutectic mixture that has a L-menthol and L-menthyl lactate weight ratio of 3:7 to 7:3; a concentration of 12-hydroxystearic acid from 0.1 to 10 wt%; and a melting point below 25 degrees Celsius. A further example of such a preferred eutectic mixture is a eutectic mixture that has a L-menthol and L-menthyl lactate weight ratio of 3:7 to 7:3; a concentration of 12-hydroxystearic acid from 1 to 7 wt%; and a melting point below 20 degrees Celsius. Yet a further example of such a preferred eutectic mixture is a eutectic mixture that has a L-menthol and L-menthyl lactate weight ratio of 3:7 to 7:3; a concentration of 12-hydroxystearic acid from 0.5 to 5 wt%; and a melting point below 20 degrees Celsius.

### 12-HSA

The inventive ternary eutectic mixtures include 12-HSA. The 12-HSA is used in its acid form as this will form a ternary eutectic mixture with L-menthol and L-menthyl lactate. A further advantage is that 12-HSA in its acid form may have a better bioavailability, especially so if part of a eutectic mixture in which it is liquid at human skin temperature (i.e. about 37 degrees Celsius) or room temperature. As with other fatty acids the apparent pKa for 12-HSA is expected to be greater than 8. Preferably, the active eutectic mixtures of the invention have a pH between 3 and 4.

### L-menthol and L-menthyl lactate

Menthol is a waxy, crystalline substance that is clear or white in color and solid at room temperature. It is used in personal care products like skin care and hair care products as it is known to soothe and cool the skin.

Binary eutectic mixtures of menthol and menthyl lactate have been described in US6897195. Eutectic compositions have a narrow formulation space. Once a combination of two ingredients has been found to form a eutectic composition there is little to no tolerance for replacing the specific ingredients or adding further ingredients without destroying the eutectic characteristics thereof.

It was surprisingly found that a specific combination of L-menthol, L-menthyl lactate and 12-hydroxystearic acid forms a ternary eutectic mixture.

The active eutectic mixtures of the present invention can be suitably made using conventional techniques and can for example be made by simply mixing the ingredients in the required amounts by grinding them until a homogenous mixture is obtained.

### Personal care composition

Personal care compositions of the invention comprise:
i) the active eutectic mixture according the invention, and
ii) a cosmetically acceptable carrier.

The personal care compositions of the invention may comprise up to 20 wt% of the eutectic mixture calculated on total personal care composition, more preferably up to 10 wt%, even more preferably up to 7 wt%, still more preferably up to 5 wt% and still even more preferably up to 2 wt%. Preferably the personal care composition comprises at least 0.1 wt% of the eutectic mixture calculated on total personal care composition, more preferably at least 0.5 wt%, even more preferably at least 1 wt%, and still more preferably at least 2 wt%. Preferred amounts of the eutectic mixture in the personal care composition are from 0.1 to 20 wt%, more preferably from 0.2 to 10 wt%, even more preferably from 0.3 to 7 wt%, still more preferably from 0.4 to 5 wt% and still even more preferably from 0.5 to 2 wt%.

The personal care composition preferably comprises from 0.005 to 15 wt% of 12-hydroxystearic acid calculated on total personal care composition, more preferably from 0.01 to 12 wt%, still more preferably from 0.1 to 10 wt%, even more preferably from 0.5 to 8 wt%, and still even more preferably from 1 to 5 wt%. Typical amounts of 12-hydroxy stearic acid in the personal care composition according to the invention are from 0.005 to 10 wt%, from 0.005 to 2 wt%, and 0.01 to 1 wt%. The amounts of 12-HSA are not meant to be included within the surfactants amounts herein.

As with other fatty acids the apparent pKa for 12-HSA is expected to be greater than 8. At the pKa, the fatty acid will exist as 50% soap and 50% acid. Therefore, preferably the pH of the personal care compositions is less than about 8, more preferably is in the range of from 3.5 to 8.0, even more preferably is from 5 to 7.8 and still more preferably is from 5.5 to 7.

It is to be understood that any one of the components making up the active eutectic mixture in the personal care composition may also be added to the personal care composition in addition to the components present as part of the eutectic mixture. The amount given for the component is the total amount of the same in the personal care composition. Preferably the components of the active eutectic mixture in the personal care composition are only present as part of the active eutectic mixture.

It should be known that commercially acceptable and conventional vehicles may be used acting as diluents, dispersants and/or carriers for the active eutectic mixture of the invention and additives described herein and for any other optional but often preferred additives. Therefore, the cosmetically acceptable carrier suitable for use in this invention may be aqueous-based, anhydrous or an emulsion whereby a water-in-oil or oil-in-water emulsion is generally preferred. If the use of water is desired, water typically makes up the balance of the cosmetic composition, and preferably makes up from 5 to 99 wt%, and most preferably from 40 to 80 wt% on total personal care composition including all ranges subsumed therein.

In addition to water, organic solvents may be optionally included within the compositions of the present invention. Illustrative and non-limiting examples of the types of organic solvents suitable for use in the present invention include alkanols like ethyl and isopropyl alcohol, mixtures thereof or the like.

Other optional additives suitable for use include ester oils like isopropyl myristate, cetyl myristate, 2-octyldodecyl myristate, avocado oil, almond oil, olive oil, neopentylglycol dicaprate, mixtures thereof or the like. Typically such ester oils assist in emulsifying the personal care composition of this invention, and an effective amount is often used to yield a stable, and most preferably water-in-oil emulsion

Emollients may also be used, if desired, within the personal care composition of the present invention. Alcohols like 1-hexadecanol (i.e. cetyl alcohol) and phenoxyethanol are often desired as are the emollients generally classified as silicone oils and synthetic esters. Silicone oils suitable for use include cyclic or linear polydimethylsiloxanes containing from 3 to 9, preferably from 4 to 5 silicon atoms. Non-volatile silicone oils useful as an emollient material in the inventive personal care composition described herein include polyalkyl siloxanes, polyalkylaryl siloxanes and polyether siloxane copolymers. The essentially non-volatile polyalkyl siloxanes useful herein include, for example, polydimethylsiloxanes.

The ester emollients that may optionally be used are:
(1) Alkenyl or alkyl esters of fatty acids having 10 to 20 carbon atoms. Examples thereof include isoarachidyl neopentanoate, isononyl isonanonoate, oleyl myristate, oleyl stearate and oleyl oleate.
(2) Ether-esters such as fatty acid esters of ethoxylated fatty alcohols.
(3) Polyhydric alcohol esters. Ethylene glycol mono- and di-fatty acid esters, diethylene glycol mono-and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, stearyl stearate and arachidyl behenate.
(5) Sterols esters, of which cholesterol fatty acid esters are examples.

Emollients, when used, typically make up from 0.1 to 50 wt% on total composition including all ranges subsumed therein.

Fatty acids having from 10 to 30 carbon atoms may also be included as cosmetically acceptable carriers within the composition of the present invention. Illustrative examples of such fatty acids include pelargonic, lauric, myristic, palmitic, stearic, isostearic, oleic, linoleic, arachidic, behenic or erucic acid and mixtures thereof. These fatty acids are in addition to the 12-HSA that is present as part of the active eutectic mixture.

Compounds that are believed to enhance skin penetration, like dimethyl sulfoxide, may also be used as an optional carrier.

Humectants of the polyhydric alcohol type may also be employed in the personal care compositions of this invention. The humectant often aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The amount of humectant may range anywhere from 0.2 to 25 wt%, and preferably from 0.5 to 15 wt% on total cosmetic composition and including all ranges subsumed therein.

Thickeners may also be utilized as part of the cosmetically acceptable carrier in the personal care compositions of the present invention. Typical thickeners include crosslinked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0 to 5 wt%, usually from 0.001 to 1 wt%, optimally from 0.01 to 0.5 wt% on total composition.

Collectively, the water, solvents, silicones, esters, fatty acids, humectants and/or thickeners will constitute the cosmetically acceptable carrier in amounts from 1 to 99.9 wt%, preferably from 80 to 99 wt% on total composition.

Surfactants may also be present in cosmetic compositions of the present invention. Total concentration of the surfactant will range from 0 to 40 wt%, and preferably from 0 to 20 wt% optimally from 0 to 5 wt% on total composition. The surfactant may be selected from the group consisting of anionic, nonionic, cationic and amphoteric actives. Particularly preferred nonionic surfactants are those with a C10-C20 fatty alcohol or acid hydrophobe condensed with from 2 to 100 moles of ethylene oxide or propylene oxide per mole of hydrophobe; C2-C10 alkyl phenols condensed with from 2 to 20 moles of alkylene oxide; mono- and di- fatty acid esters of ethylene glycol; fatty acid monoglyceride; sorbitan, mono- and di- C8-C20 fatty acids; block copolymers (ethylene oxide/propylene oxide); and polyoxyethylene sorbitan as well as combinations thereof. Alkyl polyglycosides and saccharide fatty amides (e. g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C8-C20 acyl isothionates, acyl glutamates, C8-C20 alkyl ether phosphates and combinations thereof.

Perfumes may be used in the cosmetic composition of this invention. Illustrative nonlimiting examples of the types of perfumes that may be used include those comprising terpenes and terpene derivatives like those described in Bauer, K., et al., Common Fragrance and Flavor Materials, VCH Publishers (1990). Illustrative yet non-limiting examples of the types of fragrances that may be used in this invention include myrcene, dihydromyrenol, citral, tagetone, cis-geranic acid, citronellic acid, or cis-geranic acid nitrile, mixtures thereof or the like. Preferably, the amount of fragrance employed in the cosmetic composition of this invention is in the range from 0 to 10 wt%, more preferably 0.00001 to 5 wt %, most preferably 0.0001 to 2 wt%.

Various types of optional additional active ingredients may be used in the cosmetic compositions of the present invention. Actives are defined as skin benefit agents other than emollients and other than ingredients that merely improve the physical characteristics of the composition. Although not limited to this category, general examples include talcs and silicas, as well as alpha-hydroxy acids, beta-hydroxy acids, peroxides, zinc salts and sunscreens.

Beta-hydroxy acids include salicylic acid, for example. Zinc pyrithione is an example of the zinc salts useful in the personal care composition of the present invention.

Preferably the personal care composition of the invention comprises a sunscreen. Preferably selected from organic UVA sunscreen, organic UVB sunscreen, inorganic sunscreen and combinations thereof. Particularly preferred are such materials as ethylhexyl p-methoxycinnamate, available as Parsol MCX®, Avobenzene available as Parsol 1789®, and benzophenone-3 also known as Oxybenzone. Inorganic sunscreen actives may be employed such as microfine (1 to 100 nm) titanium dioxide and zinc oxide. Amounts of the sunscreen agents when present may generally range from 0.1 to 30 wt%, preferably from 2 to 20 wt%, optimally from 4 to 10 wt% on total composition.

Many personal care compositions, especially those containing water, should be protected against the growth of potentially harmful microorganisms. Anti-microbial compounds, such as triclosan, and preservatives are typically necessary. Suitable preservatives include alkyl esters of p-hydroxybenzoic acid, hydantoin derivatives, propionate salts and a variety of quaternary ammonium compounds. Particularly preferred preservatives of this invention are methyl paraben, propyl paraben, phenoxyethanol and benzyl alcohol. Preservatives will usually be employed in amounts ranging from 0.1 to 2 wt% on total composition.

Preferably the personal care composition of the invention comprises a vitamin. Illustrative vitamins are Vitamin A (retinol), Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 to 10 wt%, preferably from 0.01 to 1 wt%, optimally from 0.1 to 0.5 wt% on total composition. Preferably the personal care composition of the invention comprises a vitamin, wherein the vitamin comprises niacinamide.

Personal care compositions of the present invention are preferably selected from leave-on skin lotions and creams, shampoos, hair conditioners, shower gels, toilette bars, antiperspirants, deodorants, shave creams, lipsticks, foundations and sunscreen lotions.

Personal care compositions of the present invention may be in any form. These forms may include lotions, creams, roll-on formulations, sticks, mousses, aerosol and non-aerosol sprays and fabric (e.g. nonwoven textile)-applied formulations.

The personal care compositions of the present invention preferably are non-solid. Essentially, the "non-solidness" of the composition means that the viscosity of the compositions, e.g. as measured using a Brookfield DV-I + viscometer (20RPM, RV6, 30 seconds), in general is in the range of from 1 Pas to 500Pas, preferably from 1 Pas to 200Pas, more preferably from 2Pas to 100Pas, most preferably from 3Pas to 50Pas at 20 degrees Celsius.

The personal care composition of the invention is especially suited for use in hair and (as part of that)/or as scalp care. In addition to the scalp skin benefits that may be delivered by 12-HSA, the cooling effect of the menthol that is part of the active eutectic mixture is much appreciated by users of such products. Hair and/or scalp care compositions may be rinse-off products or leave-on products. Leave-on products are intended not to be rinsed off the hair and/or the scalp of the user immediately after use (i.e. within at least the first two hours, preferably at least four hours, after application of the composition). Leave-on products include, for example, lotions, creams and hair oils that are intended for topical application to the hair and/or the scalp. Rinse-off products are intended to be substantially rinsed off the hair and/or the scalp of the user with water after use. Rinse-off compositions include shampoos and hair conditioners, as well as hair and/or scalp treatment products which are intended to be left on the hair and/or scalp for up to half an hour, preferably 5 minutes, before being rinsed off. The personal care composition is preferably a shampoo (i.e. a rinse-off hair personal care product) or hair conditioner (i.e. a leave-on hair personal care product).

Even though the skin benefits of 12-HSA may also be delivered as part of a rinse off personal care composition, preferred compositions of the invention are leave-on compositions (i.e. personal care compositions that are meant to be left on after application by the consumer to the appropriate area of the human body like skin and/or hair). Preferred compositions of the present invention are intended to be applied to remain on the skin. These leave-on compositions are to be distinguished from compositions which are applied to the skin and subsequently removed either by washing, rinsing, wiping, or the like either after or during the application of the product. Surfactants typically used for rinse-off compositions have physico-chemical properties giving them the ability to generate foam/lather in-use with ease of rinse; they can consist of mixtures of anionic, cationic, amphoteric, and nonionic. Surfactants used in leave-on compositions on the other hand are not required to have such properties. Rather, as leave-on compositions are not intended to be rinsed-off they need to be non-irritating and therefore it would be necessary to minimize the total level of surfactant, and particularly the total level of anionic surfactant in leave-on compositions. Therefore, the preferred leave-on personal care compositions of the present invention contain, with respect to surfactants, predominantly nonionic surfactants. The anionic surfactants are present in an amount of at most 5 wt% on total composition, preferably from 0.01 to 4 wt%, more preferably from 0.01 to 3 wt%, most preferably from 0.01 to 2 wt% and optimally are substantially absent (e.g. less than 1 wt%, preferably less than 0.1 wt%, or even less than 0.01 wt%). Salts of 12-HSA are not considered anionic surfactants herein. The total level of surfactant in the inventive compositions is preferably no more than 10 wt% on total composition, more preferably below 8 wt%, most preferably at most 5 wt%.

A wide variety of packaging can be employed to store and deliver the personal care compositions. Packaging is often dependent upon the type of personal care end-use. For instance, leave-on skin lotions and creams, shampoos, hair conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered as a stick composition formulation in a container with propel-repel mechanism where the stick moves on a platform towards a dispensing orifice. Metallic cans pressurized by a propellant and having a spray nozzle serve as packaging for antiperspirants, shave creams and other sprayable personal care products. Toilet bars may have packaging constituted by a cellulosic or plastic wrapper or within a cardboard box or even encompassed by a shrink wrap plastic film.

### Method for preparing the personal care composition

It was found that the eutectic mixtures of the invention can be formulated into personal care compositions without losing their eutectic properties, i.e. the lower melting point. This can be achieved by separately making the eutectic mixture and combine this pre-formed eutectic mixture with the other ingredients of the personal care composition.

The invention thus also relates to a method for preparing the personal care composition of the present invention comprising the step of mixing the pre-formed active eutectic mixture of L-menthol, L-menthyl lactate and 12-hydroxystearic acid with part or all of the remaining ingredients of the personal care composition.

This results in a product that comprises 12-HSA with a lower melting point (as part of the eutectic mixture) but also provides a product that is storage stable in that for example the 12-HSA does not re-crystallize, or at least at a slower pace and/or only partially. These advantages are absent or at least less pronounced when the 12-HSA is formulated into a personal care composition without L-menthol and L-menthyl lactate or if the L-menthol, L-menthyl lactate and 12-HSA are added as separate ingredients (i.e. not as a pre-formed eutectic mixture).

The invention also concerns the personal care composition of the invention obtainable by the method for preparing the personal care composition.

The invention further concerns a method of applying to human skin the personal care composition of the invention.

The invention is now illustrated by the following non-limiting examples.

### Examples

### Melting point (DSC curve)

A Mettler DSC 823 was used for the thermal analyses to measure the melting point. For the "pure" mixtures, i.e. only containing L-menthol, L-menthyl lactate and 12-HSA, the temperature range and scanning speed were set to -70 to 100 °C and 2°C/min respectively.

For personal care compositions comprising the eutectic mixture the temperature range and scanning speed were set to 0 to 120°C and 10°C/min respectively. The measurements were performed with an accuracy of ±0.01 °C. The measurements were executed at atmospheric pressure in a dynamic nitrogen atmosphere. The samples were introduced into an aluminum crucible (40 µl) or medium pressure crucible (120 µl). The pan was sealed and introduced at room temperature into the DSC. A special pan, included in the standard accessories of the Mettler equipment, was used to perform the temperature and enthalpy calibration.

### EXAMPLE 1

Mixtures of L-menthol, L-menthyl lactate and 12-HSA where prepared as follows, using the ingredients as in Table 1 and the amounts mentioned in Tables 2A and 2B.

The three components L-menthol, L-menthyl lactate and 12-HSA were weighed and then mixed by grinding in a mortar and pestle until a homogenous mixture was obtained. Mixtures that started to melt at room temperature were mixed by grinding until a transparent liquid was formed.

**Table 1, Ingredients of ternary eutectic mixtures**

| **Ingredient** | **Purity** | **CAS#** | **Supplier** |
|---|---|---|---|
| 12-HSA | 95% | 106-14-9 | Alfa Aesar |
| L-menthol | 99.5% | 2216-51-5 | Damas-beta |
| L-menthyl lactate | 99% | 69-72-7 | Sigma |

The melting point of these mixtures was measured and the results are shown in Tables 2A and 2B. It is clearly shown that specific mixtures have a lower melting temperature. The lowest melting point of about -52 degrees Celsius was determined for a eutectic composition of L-menthol and L-menthyl lactate in a weight ratio of 4:6 and a 12-HSA concentration of 1 wt% with respect to the total weight of the three components together. This composition is believed to be close to the "true" eutectic composition of this ternary system.

**Table 2A, Melting point of mixtures of L-menthol, L-menthyl lactate and 12-HSA**

| **The weight ratio of L-menthol to L-menthyl lactate** | **Concentration of 12-HSA (wt%)** | | | | |
|---|---|---|---|---|---|
| | 0 | 1 | 3 | 7 | 10 |
| | Melting point of L-menthol, L-menthyl lactate and 12-HSA (°C) | | | | |
| 1:9 | 38 | 40.66 | - | - | 38.95 |
| 2:8 | 26.5 | 33.49 | - | - | 32.83 |
| 3:7 | 19 | 26.12 | - | - | 33.48 |
| 4:6 | 15 | -52.25 | 15.17 | 22.5 | 32.13 |
| 5:5 | 8.2 | 9.8 | 1.58 | 21.56 | 31.03 |
| 6:4 | 15 | 18.2 | 8.32 | 17.02 | 30.79 |
| 7:3 | 20 | 24.73 | - | - | 22.18 |
| 8:2 | 28 | 29.87 | - | - | 25.75 |
| 9:1 | 39 | 39.33 | - | - | 32.11 |

**Table 2B, Melting point of mixtures of L-menthol, L-menthyl lactate and 12-HSA**

| **The weight ratio of L-menthol to L-menthyl lactate** | **Concentration of 12-HSA (wt%)** | | | | |
|---|---|---|---|---|---|
| | 15 | 20 | 30 | 40 | 50 |
| | Melting point of L-menthol, L-menthyl lactate and 12-HSA (°C) | | | | |
| 1:9 | 50.64 | 51.35 | 56.61 | 61.23 | 64.92 |
| 2:8 | 44.79 | 48.81 | 54.62 | 59.85 | 64.34 |
| 3:7 | 42.69 | 46.52 | 53.61 | 58.92 | 63.49 |
| 4:6 | 41.43 | 43.03 | 52.03 | 57.59 | 61.77 |
| 5:5 | 37.84 | 42.88 | 51.08 | 55.39 | 61.06 |
| 6:4 | 37.08 | 40.76 | 51.11 | 56.38 | 60.3 |
| 7:3 | 34.37 | 40.78 | 50.07 | 55.66 | 60.08 |
| 8:2 | 25.86 | 41.43 | 46.55 | 49.5 | 59.61 |
| 9:1 | 30.92 | 38.29 | 47.61 | 52.7 | 59.04 |

### EXAMPLE 2

A personal care composition (PCC 1) according to Table 3A comprising a pre-formed mixture of L-menthol, L-menthyl lactate and 12-HSA was prepared using the following protocol.
1. Phase A was prepared by mixing the ingredients and heating the mixture to 70 degrees Celsius to dissolve the ingredients.
2. Phase B was prepared by mixing sodium polyacrylate and 1,3-butylene glycol and adding them to Phase A. Glycerin was used to flush the beaker and decanted into phase A. The same was repeated with water.
3. Phase C was prepared by mixing the ingredients at 75 degrees Celsius under manual stirring until the phase was transparent.
4. Phase D was prepared by mixing the ingredients in a mortar and pestle until a transparent liquid was obtained. This liquid was added to Phase C.
5. The ingredients of phase E and F were mixed separately and added at 75 degrees Celsius to the mixture obtained after step 4) and stirred manually.
6. Phase G was added to the mixture obtained after step 5) and the mixture was homogenized by mixing for 3 minutes with a Silverson mixer at speed setting 4.
7. The mixture obtained after step 6) was added to the combined phases A+B obtained after step 2). Phase H was used to flush the beaker and completely transfer the mixture from step 6) into the combined phases A+B from step 2). The resulting mixture was homogenized using a Silverson mixer at setting 7 for 10 min until a homogenous mixture is obtained.
8. Sodiumpolyacrylate and 1,3-butylene glycol were mixed and heated to 75 degrees Celsius until dissolved to form phase I. The resulting liquid was added to the main mix from step 7) to increase the viscosity. Water was used to flush the beaker. After phase I had been completely transferred the mixture was homogenized using a Silverson mixer at speed setting 7 for 5min. The mixture was then stirred until it had cooled down to below 45 Degrees Celsius.
9. The ingredients of phase J were mixed and added to the main mix obtained after step 8) after it had cooled down to below 35 degrees Celsius. The resulting mixture was homogenized for 3min using a Silverson mixer at speed setting 7.
10. The ingredients of phase K are added to the mixture obtained after step 9 and homogenized for 5min using a Silverson at speed stetting 7.

**Table 3A, Water based personal care composition PCC 1 (wt%)**

| **Phase** | **Ingredient** | **PCC 1** |
|---|---|---|
| A | water | 39.78 |
| | EDTA Na₂ | 0.05 |
| | methyl Paraben | 0.20 |
| | sodium hydrogen N-(1-oxooctadecyl)-L-glutamate | 0.50 |
| B | sodium polyacrylate | 0.35 |
| | 1,3-Butylene glycol | 1.00 |
| | glycerin (Flush) | 3.00 |
| | water | 2.00 |
| C | propyl paraben | 0.10 |
| | stearyl heptanoate | 1.00 |
| | cetearyl alcohol | 2.40 |
| | dicaprylyl carbonate | 4.00 |
| | Hydrogenated Polydecene (Puresyn 6, ex ExxonMobile) | 3.00 |
| D | L-menthol (ex Symrise) | 3.96 |
| | L-menthyl lactate (ex Symrise) | 5.94 |
| | 12-HSA (ex Biotor) | 0.10 |
| E | sucrose polystearate (Emulgade SUCRO, ex Cognis) | 1.00 |
| | DC 200/200cst | 2.00 |
| F | Parsol 1789 | 1.95 |
| | Parsol MCX | 6.00 |
| G | Titanium Dioxide (SA-TR-10, ex Miyoshi Kasei) | 0.60 |
| H | capric triglycerides (GTCC (Flush), myritol 318; ex Croda) | 2.00 |
| I | sodium polyacrylate (Covacryl MV 60, ex Sensient) | 0.20 |
| | 1,3-butylene glycol | 1.00 |
| | water | 2.00 |
| J | water | 12.00 |
| | vitamin B3 | 3.00 |
| K | Annmonium Lactate (L) (70%) | 0.071 |
| | Jasmin silk 3457M | 0.40 |
| | Phenoxyethanol | 0.4% |

| | | |
|---|---|---|
| Parsol 1789: Butyl Methoxydibenzoylmethane Parsol MCX: Ethylhexyl Methoxycinnamate | | |

### EXAMPLE 3

Personal care compositions according to Table 3B comprising were prepared using the following protocol. PCC 2 comprises 12-HSA, but no L-menthol and L-menthyl lactate. In PCC 3, L-menthol, L-menthyl lactate and 12-HSA were added separately (i.e. not added as a pre-formed eutectic mixture).
1. Phase A was prepared by mixing the ingredients and heating the mixture to 70 degrees Celsius to dissolve the ingredients.
2. Phase B was prepared by mixing sodium polyacrylate and 1,3-butylene glycol and adding them to Phase A. Glycerine was used to flush the beaker and decanted into phase A. The same was repeated with water.
3. Phase C was prepared by was prepared by mixing the ingredients at 75 degrees Celsius under manual stirring until the phase was transparent. For PCC 3, 12-HSA, L-menthol and L-menthyl lactate were added to the dispersion one by one as the last ingredients.
4. The ingredients of phase D and E were mixed separately and then added to the mixture obtained in step 3).
5. Phase F was then added to the mixture obtained after step 4) and the resulting dispersion was homogenized using a Silverson mixer at speed stetting 4.
6. The mixture obtained after step 5) was then added to the combined phases A and B obtained after step 2). Phase G was used to flush the beaker and completely transfer the mixture from step 5) into the combined phases A and B from step 2). The resulting mixture was homogenized using a Silverson mixer at setting 7 for 10 min until a homogenous mixture is obtained.
7. Sodium polyacrylate 1,3-butylene glycol were mixed and heated to 75 degrees Celsius until dissolved to form phase H. The resulting liquid was added into the main mix from step 6) to increase the viscosity. Water was used to flush the beaker. After phase H had been completely transferred the mixture was homogenized using a Silverson mixer at speed setting 7 for 5min. The mixture was then stirred until it had cooled down to below 45 degrees Celsius.
8. The ingredients of phase I were mixed and added to the main mix obtained after step 7) after it had cooled down to below 35 degrees Celsius. The resulting mixture was homogenized for 3min using a Silverson mixer at speed setting 7.
9. The ingredients of phase J were added to the mixture obtained after step 8 and homogenized for 5min using a Silverson at speed stetting 7.

**Table 3B, Water based personal care compositions PCC 2 and PCC 3 (wt%)**

| **Phase** | **Ingredients** | **PCC 2** | **PCC 3** |
|---|---|---|---|
| A | water | 49.68 | 39.78 |
| | EDTA Na₂ | 0.05 | 0.05 |
| | methyl paraben | 0.20 | 0.20 |
| | sodium hydrogen N-(1-oxooctadecyl)-L-glutamate | 0.50 | 0.50 |
| B | Sodium Polyacrylate | 0.35 | 0.35 |
| | 1,3-butylene glycol | 1.00 | 1.00 |
| | glycerin (Flush) | 3.00 | 3.00 |
| | water | 2.00 | 2.00 |
| C | propyl paraben | 0.10 | 0.10 |
| | stearyl heptanoate | 1.00 | 1.00 |
| | cetearyl alcohol | 2.40 | 2.40 |
| | dicaprylyl carbonate | 4.00 | 4.00 |
| | Hydrogenated Polydecene (Puresyn 6, ex Exxon Mobile) | 3.00 | 3.00 |
| | L-menthol (ex Symrise) | - | 3.96 |
| | L-menthyl lactate (ex Symrise) | - | 5.94 |
| | 12-HSA (ex Biotor) | 0.10 | 0.10 |
| D | sucrose polystearate (Emulgade SUCRO, ex Cognis) | 1.00 | 1.00 |
| | DC 200/200cst | 2.00 | 2.00 |
| E | Parsol 1789 | 1.95 | 1.95 |
| | Parsol MCX | 6.00 | 6.00 |
| F | Titanium Dioxide (SA-TR-10, ex Miyoshi Kasei) | 0.60 | 0.60 |
| G | Capric Triglycerides (GTCC (Flush), myritol 318; ex Croda) | 2.00 | 2.00 |
| H | sodium polyacrylate (covacryl MV 60, ex Sensient) | 0.20 | 0.20 |
| | 1,3-butylene glycol | 1.00 | 1.00 |
| | water | 2.00 | 2.00 |
| I | water | 12.00 | 12.00 |
| | vitamin B3 | 3.00 | 3.00 |
| J | Annmonium Lactate (L) (70%) | 0.071 | 0.071 |
| | Jasmin silk 3457M | 0.40 | 0.40 |
| | phenoxyethanol | 0.40 | 0.40 |

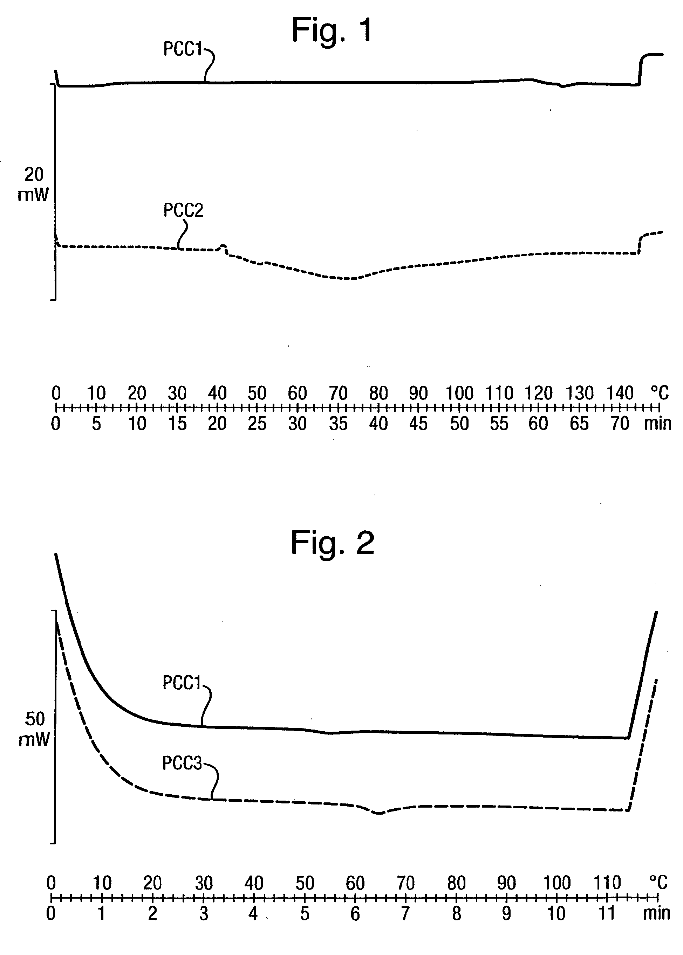

The DSC curves for PCC 1 and PCC 2 were measured (medium pressure crucible (120 µl)) and shown in Figure 1.

No exothermic peak was observed for PCC 1, indicating that no crystals were present in the formulation according to the invention containing the active ternary mixture. PCC 2, only comprising 12-HSA, showed an exothermic peak around 80 °C, which corresponds to the melting temperature of crystalline 12-HSA.

The DSC curves for PCC 1 and PCC 3 were measured after storage for one week at 50 degrees Celsius for (medium pressure crucible (120 µl)) and shown in Figure 2. There is still no exothermic peak of 12-HSA in PCC 1, but there is an exothermic peak in PCC 3. This shows that the active eutectic mixture according to the invention can inhibit the crystallization of 12-HSA.

## Claims

1. Active eutectic mixture of L-menthol, L-menthyl lactate and 12-hydroxystearic acid, wherein the weight ratio of L-menthol to L-menthyl lactate is from 2:8 to 9:1; the concentration of 12-hydroxystearic acid calculated on total weight of the eutectic mixture is from 0.1 to 17 wt%; and wherein the eutectic mixture has a melting point below 40 degrees Celsius.

2. Mixture according to claim 1 wherein the weight ratio of L-menthol to L-menthyl lactate is from 2:8 to 8:2, more preferably from 3:7 to 7:3, even more preferably from 3:7 to 6:4, still more preferably from 3:7 to 5:5 and even still more preferably from 4:6 to 5:5.

3. Mixture according to claim 1 or claim 2 wherein the concentration of 12-hydroxystearic acid calculated on total weight of the eutectic mixture is from 0.1 to 15 wt%, more preferably from 0.1 to 10 wt%, even more preferably from 0.2 to 5 wt% and still more preferably from 0.5 to 3 wt%.

4. Mixture according to any one of claims 1 to 3 wherein the mixture has a melting point below 37 degrees Celsius, more preferably below 30 degrees Celsius, even more preferably below 25 degrees Celsius, still more preferably below 20 degrees Celsius, even still more preferably below 15 degrees Celsius and more so preferably below 10 degrees Celsius.

5. Personal care composition comprising
i) the active eutectic mixture according to any one of claims 1 to 4, and
ii) a cosmetically acceptable carrier.

6. Composition according to claim 5 comprising up to 20 wt% of the eutectic mixture calculated on total personal care composition, more preferably up to 10 wt%, even more preferably up to 7 wt%, still more preferably up to 5 wt% and still even more preferably up to 2 wt%.

7. Composition according to claim 5 or claim 6 comprising from 0.005 to 15 wt% of 12-hydroxystearic acid, more preferably from 0.01 to 12 wt%, even more preferably from 0.1 to 10 wt%, still more preferably from 0.5 to 8 wt%, and even still more preferably from 1 to 5 wt%.

8. Composition according to any one of claims 5 to 7 further comprising a sunscreen, preferably selected from organic UVA sunscreen, organic UVB sunscreen, inorganic sunscreen and combinations thereof.

9. Composition according to any one of claims 5 to 8 further comprising a vitamin, preferably the vitamin comprising niacinamide.

10. Composition according to any one of claims 5 to 9 which is selected from leave-on skin lotions and creams, shampoos, hair conditioners, shower gels, toilette bars, antiperspirants, deodorants, shave creams, lipsticks, foundations and sunscreen lotions.

11. Composition according to any one of claims 5 to 10 which is a shampoo or hair conditioner.

12. Method for preparing a personal care composition according to any one of claims 5 to 11 comprising the step of mixing the pre-formed active eutectic mixture of L-menthol, L-menthyl lactate and 12-hydroxystearic acid with part or all of the remaining ingredients of the personal care composition.

13. Personal care composition according to any one of claims 5 to 11 obtainable by the method of claim 12.

14. Method of applying to human skin the personal care composition according to any one of claims 5 to 11 or claim 13.

## Patentansprüche

1. Aktive eutektische Mischung von L-Menthol, L-Menthyllactat und 12-Hydroxystearinsäure, wobei das Gewichtsverhältnis von L-Menthol zu L-Menthyllactat von 2:8 bis 9:1 liegt, die Konzentration der 12-Hydroxystearinsäure, berechnet auf Gesamtgewicht der eutektischen Mischung, von 0,1 bis 17 Gew.-% ist und worin die eutektische Mischung einen Schmelzpunkt unterhalb 40 Grad Celsius aufweist.

2. Mischung nach Anspruch 1, wobei das Gewichtsverhältnis von L-Menthol zu L-Menthyllactat von 2:8 bis 8:2, bevorzugter von 3:7 bis 7:3, sogar bevorzugter von 3:7 bis 6:4, noch bevorzugter von 3:7 bis 5:5 und sogar noch bevorzugter von 4:6 bis 5:5 liegt.

3. Mischung nach Anspruch 1 oder 2, wobei die Konzentration der 12-Hydroxystearinsäure, berechnet auf Gesamtgewicht der eutektischen Mischung, von 0,1 bis 15 Gew.-%, bevorzugter von 0,1 bis 10 Gew.-%, sogar bevorzugter von 0,2 bis 5 Gew.-% und sogar noch bevorzugter von 0,5 bis 3 Gew.-% beträgt.

4. Mischung nach einem der Ansprüche 1 bis 3, wobei die Mischung einen Schmelzpunkt unter 37 Grad Celsius, bevorzugter unter 30 Grad Celsius, sogar bevorzugter unter 25 Grad Celsius, noch bevorzugter unter 20 Grad Celsius, sogar noch bevorzugter unter 15 Grad Celsius und umso bevorzugter unter 10 Grad Celsius aufweist.

5. Körperpflegezusammensetzung, umfassend
i) die aktive eutektische Mischung gemäß irgendeinem der Ansprüche 1 bis 4,
und
ii) einen kosmetisch akzeptablen Träger.

6. Zusammensetzung gemäß Anspruch 5, umfassend bis zu 20 Gew.-% eutektische Mischung, berechnet auf der gesamten Körperpflegezusammensetzung, bevorzugter bis zu 10 Gew.-%, sogar bevorzugter bis zu 7 Gew.-%, noch bevorzugter bis zu 5 Gew.-% und sogar noch bevorzugter bis zu 2 Gew.-%.

7. Zusammensetzung gemäß Anspruch 5 oder 6, umfassend von 0,005 bis 15 Gew.-% 12-Hydroxystearinsäure, bevorzugter von 0,01 bis 12 Gew.-%, sogar bevorzugter von 0,1 bis 10 Gew.-%, noch bevorzugter von 0,5 bis 8 Gew.-% und sogar noch bevorzugter von 1 bis 5 Gew.-%.

8. Zusammensetzung gemäß irgendeinem der Ansprüche 5 bis 7, des Weiteren umfassend einen Sonnenschutz, vorzugsweise ausgewählt unter organischem UVA-Sonnenschutz, organischem UVB-Sonnenschutz, anorganischem Sonnenschutz und Kombinationen davon.

9. Zusammensetzung gemäß irgendeinem der Ansprüche 5 bis 8, des Weiteren umfassend ein Vitamin, vorzugsweise das Vitamin, das Nicotinamid umfasst.

10. Zusammensetzung gemäß irgendeinem der Ansprüche 5 bis 9, die ausgewählt ist unter Leave-on-Hautlotionen und Cremes, Shampoos, Haarkonditioniermitteln, Duschgels, Toilettenstäben, Antischweißmitteln, Deodorants, Rasiercremes, Lippenstiften, Foundations und Sonnenschutz-Lotionen.

11. Zusammensetzung gemäß irgendeinem der Ansprüche 5 bis 10, die ein Shampoo oder ein Haarkonditioniermittel darstellt.

12. Verfahren zur Herstellung einer Körperpflegezusammensetzung gemäß irgendeinem der Ansprüche 5 bis 11, umfassend die Schritte des Mischens des vorgeformten aktiven eutektischen Gemisches von L-Menthol, L-Menthyllactat und 12-Hydroxystearinsäure mit einem Teil oder sämtlichen verbleibenden Bestandteilen der Hautpflegezusammensetzung.

13. Körperpflegezusammensetzung gemäß irgendeinem der Ansprüche 5 bis 11, erhältlich durch das Verfahren des Anspruchs 12.

14. Verfahren zum Auftragen der Hautpflegezusammensetzung gemäß irgendeinem der Ansprüche 5 bis 11 oder 13 auf menschliche Haut.

## Revendications

1. Mélange eutectique actif de L-menthol, de lactate de L-menthyle et d'acide 12-hydroxystéarique, dans lequel le rapport massique de L-menthol à lactate de L-menthyle est de 2:8 à 9:1, la concentration d'acide 12-hydroxystéarique calculée sur une masse totale du mélange eutectique est de 0,1 à 17 % en masse ; et dans lequel le mélange eutectique présente un point de fusion inférieur à 40 degrés Celsius.

2. Mélange selon la revendication 1, dans lequel le rapport massique de L-menthol à lactate de L-menthyle est de 2:8 à 8:2, encore mieux de 3:7 à 7:3, bien mieux encore de 3:7 à 6:4, encore mieux de préférence de 3:7 à 5:5 et bien mieux encore de préférence de 4:6 à 5:5.

3. Mélange selon la revendication 1 ou la revendication 2, dans lequel la concentration d'acide 12-hydroxystéarique calculée sur une masse totale du mélange eutectique est de 0,1 à 15 % en masse, encore mieux de 0,1 à 10 % en masse, bien mieux encore de 0,2 à 5 % en masse et encore mieux de préférence de 0,5 à 3 % en masse.

4. Mélange selon l'une quelconque des revendications 1 à 3, dans lequel le mélange présente un point de fusion inférieur à 37 degrés Celsius, encore mieux inférieur à 30 degrés Celsius, bien mieux encore inférieur à 25 degrés Celsius, encore mieux de préférence inférieur à 20 degrés Celsius, bien mieux encore de préférence inférieur à 15 degrés Celsius et particulièrement de préférence inférieur à 10 degrés Celsius.

5. Composition de soins personnels comprenant
i) le mélange eutectique actif selon l'une quelconque des revendications 1 à 4, et
ii) un support cosmétiquement acceptable.

6. Composition selon la revendication 5 comprenant jusqu'à 20 % en masse du mélange eutectique calculés sur une composition de soins personnels totale, encore mieux jusqu'à 10 % en masse, bien mieux encore jusqu'à 7 % en masse, encore mieux de préférence jusqu'à 5 % en masse et bien mieux encore de préférence jusqu'à 2 % en masse.

7. Composition selon la revendication 5 ou la revendication 6 comprenant de 0,005 à 15 % en masse d'acide 12-hydroxystéarique, encore mieux de 0,01 à 12 % en masse, bien mieux encore de 0,1 à 10 % en masse, encore mieux de préférence de 0,5 à 8 % en masse, et bien mieux encore de préférence de 1 à 5 % en masse.

8. Composition selon l'une quelconque des revendications 5 à 7 comprenant de plus un écran solaire, de préférence choisi parmi un écran solaire UVA organique, un écran solaire UVB organique, un écran solaire inorganique et des combinaisons de ceux-ci.

9. Composition selon l'une quelconque des revendications 5 à 8 comprenant de plus une vitamine, la vitamine comprenant de préférence du niacinamide.

10. Composition selon l'une quelconque des revendications 5 à 9, laquelle est choisie parmi des lotions pour la peau sans rinçage et des crèmes, des shampoings, des après-shampoings, des gels douches, des savons de toilette, des antiperspirants, des déodorants, des crèmes de rasage, des rouges à lèvres, des fonds de teint et des lotions d'écran solaire.

11. Composition selon l'une quelconque des revendications 5 à 10, laquelle est un shampoing ou un après-shampoing.

12. Procédé pour la préparation d'une composition de soins personnels selon l'une quelconque des revendications 5 à 11 comprenant l'étape de mélange du mélange eutectique actif préformé de L-menthol, de lactate de L-menthyle et d'acide 12-hydroxystéarique avec une partie ou la totalité des ingrédients restants de la composition de soins personnels.

13. Composition de soins personnels selon l'une quelconque des revendications 5 à 11 pouvant être obtenue par le procédé de la revendication 12.

14. Procédé d'application à de la peau humaine de la composition de soins personnels selon l'une quelconque des revendications 5 à 11 ou la revendication 13.
